# EUROPEAN PATENT SPECIFICATION

(11) **EP 3 525 706 B1**
(45) Date of publication and mention of the grant of the patent: **16.06.2021**
(21) Application number: 17794901.3
(22) Date of filing: 16.10.2017
(51) Int. Cl.: A61B 18/02, A61B 18/00

(54) **COLD ATMOSPHERIC PLASMA TREATMENT OF ACTINIC KERATOSIS AND NON-MELANOMA SKIN CANCER**
ATMOSPHÄRISCHE KALTPLASMABEHANDLUNG AKTINISCHER KERATOSE UND NICHT-MELANOMISCHEN HAUTKREBS
TRAITMENT À PLASMA ATMOSPHÉRIQUE À FROID DE KÉRATOSE ACTINIQUE ET DU CANCER DE LA PEAU NON-MÉLANOMIQUE

(30) Priority: 15.10.2016 US 201662408765 P; 03.07.2017 US 201715640862
(43) Date of publication of application: 21.08.2019
(73) Proprietor: Wirtz, Michelle, 8008 Zürich (CH)
(72) Inventor: Wirtz, Michelle, 8008 Zürich (CH)
(74) Representative: Weickmann & Weickmann PartmbB
(86) International application number: PCT/EP2017/076354
(87) International publication number: WO 2018/069549

(56) References cited:
- WO-A1-2013/110756
- WO-A1-2015/071099
- WO-A1-2016/079742
- WO-A1-2018/026750
- WO-A1-2018/140708
- CN-U- 205 142 644
- DE-A1-102008 008 614
- DE-T2- 60 110 379
- DE-U1-202013 101 914
- US-A1- 2013 345 620
- US-A1- 2016 023 183
- US-A1- 2016 138 006
- M. WIRTZ ET AL: "Actinic keratoses treated with cold atmospheric plasma", JEADV. JOURNAL OF THE EUROPEAN ACADEMY OF DERMATOLOGY AND VENEREOLOGY., 31 July 2017 (2017-07-31), XP055437951, NL ISSN: 0926-9959, DOI: 10.1111/jdv.14465
- FRIEDMAN PETER C ET AL: "Successful treatment of actinic keratoses using nonthermal atmospheric pressure plasma: A case series", JOURNAL OF THE AMERICAN ACADEMY OF DERMATOLOGY, MOSBY, INC, US, vol. 76, no. 2, 13 January 2017 (2017-01-13), pages 349-350, XP029883044, ISSN: 0190-9622, DOI: 10.1016/J.JAAD.2016.09.004
- Adtec Europe: "Clinical Trial to investigate the potential benefits of treating Actinic Keratosis with Adtec SteriPlas plasma technology", , 5 July 2017 (2017-07-05), XP055438048, Internet Retrieved from the Internet: URL:https://adtecplasma.tumblr.com/post/16 2624109333/clinical-trial-to-investigate-t he-potential [retrieved on 2018-01-03]
- Zhitong Chen ET AL: "Cold atmospheric plasma discharged in water and its potential use in cancer therapy", Arxiv.org Preprint, 6 April 2016 (2016-04-06), pages 1-18, XP055437453, DOI: 10.1088/1361-6463/50/1/015208 Retrieved from the Internet: URL:https://arxiv.org/ftp/arxiv/papers/160 4/1604.03051.pdf [retrieved on 2017-12-21]
- GREGORY FRIDMAN ET AL: "Blood Coagulation and Living Tissue Sterilization by Floating-Electrode Dielectric Barrier Discharge in Air", PLASMA CHEMISTRY AND PLASMA PROCESSING, KLUWER ACADEMIC PUBLISHERS-PLENUM PUBLISHERS, NE, vol. 26, no. 4, 15 June 2006 (2006-06-15), pages 425-442, XP019405022, ISSN: 1572-8986, DOI: 10.1007/S11090-006-9024-4
- WENDE K ET AL: "Risk assessment of a cold argon plasma jet in respect to its mutagenicity", MUTATION RESEARCH. GENETIC TOXICOLOGY AND ENVIRONMENTAL MUTAGENESIS, ELSEVIER, AMSTERDAM, NL, vol. 798, 23 February 2016 (2016-02-23), pages 48-54, XP029447644, ISSN: 1383-5718, DOI: 10.1016/J.MRGENTOX.2016.02.003
- T. MAISCH ET AL: "Investigation of toxicity and mutagenicity of cold atmospheric argon plasma : Mutagenicity of Cold Atmospheric Plasma", ENVIRONMENTAL AND MOLECULAR MUTAGENESIS., vol. 58, no. 3, 28 March 2017 (2017-03-28) , pages 172-177, XP055724589, GB ISSN: 0893-6692, DOI: 10.1002/em.22086

## Description

The invention relates to the field of Cold Atmospheric Plasma (CAP) treatment of therapy-refractory Actinic Keratosis, wherein the cold atmospheric plasma is an argon-containing plasma.

### Description of Related Art

"Hot" (thermic) Atmospheric Plasma is well established for ablation or coagulation, but because of the temperature (> 10,000° Celsius) it is not possible to use it on physiological surfaces, including skin. Since some years "Cold" (non-thermic) Atmospheric Plasma (CAP) can be generated and used for such surfaces. CAP is a (partially or fully) ionized gas composed of multiple chemically active species, such as ions, electrons, photons, reactive oxygen and nitrogen species, and UV light. The active species of CAP are capable of inducing physical phenomena and chemical reactions on biological surfaces upon application. CAP has demonstrated significant anti-microbial efficacy in clinical trials. The common use of CAP in the medical field is e.g. surgeries, endoscopic procedures, to deactivate pathogens, stop bleeding, and actively promote wound healing by stimulation of cell proliferation of e.g. basal keratinocytes or fibroblasts. Despite this pro-proliferative effect of CAP, recent pre-clinical observations suggested an anti-tumoral potential of CAP in different malignant tumors, for example melanoma, glioma and colorectal carcinoma cells, while the biological effects and the underlying mode of action still remain unclear. Hitherto, harmful clinical effects have not been reported and *in vitro* studies did not show increased genotoxicity in cultured cells after repetitive argon plasma treatment. So far, there is no evidence for harmful clinical effects by CAP and in vitro studies could not find an increase of genotoxicity in cultured cells after treatment with plasma, such as argon plasma, up to 180 seconds (Wende et al., Mutat Res Genet Toxicol Environ Mutagen 2016; 798-799: 48-54; Maisch T, Bosserhoff AK, Unger P et al. Investigation of toxicity and mutagenicity of cold atmospheric argon plasma. Environ Mol Mutagen 2017; 58: 172-7).

US 2013/0345620 A1 provides a variety of systems, techniques and machine-readable programs for using plasmas to treat different skin conditions as well as other conditions, such as tumors, bacterial infections and the like.

DE 20 2013 101 914 U1 teaches a system for applying a plasma-modified field to a subject.

DE 601 10 379 T2 relates to repairing tissues, for example skin repair or repair or removal of internal tissue, such as gastrointestinal tract, respiratory tract, blood vessels, uterus or urethra.

Fridman et al. carried out a case series addressing treatment of actinic keratosis using non-thermal atmospheric pressure plasma (Fridman et al., J AM Acad Dermatol (2017), 76(2), 349-350).

WO 2016/079742 provides a novel configuration of plasma-generating system, enabling the use of cold plasma within a living biological cavity (e.g. suitable for endoscopic applications).

US 2016/0023183 A1 relates to the field of non-thermal plasma technology and application and also relates to means and methods for providing biological effects on microorganisms.

WO 2018/140708 A1 is directed to a system, apparatus and method for cold plasma skin resurfacing.

Fridman et al. (Plasma Chem Plasma Process, (2006), 26, 425-442) propose a method of direct treatment of living tissue that occurs at room temperature and pressure without visible or microscopic tissue damage.

WO 2018/026750 A1 discloses a method for treating actinic keratosis of tissue of a patient, the method including contacting non-thermal atmospheric pressure plasma over areas of the tissue having actinic keratosis for a time and at treatment condition effective to give rise to an at least partial amelioration of the keratosis. Prior art is also disclosed in FRIEDMAN PETER C ET AL: "Successful treatment of actinic keratoses using nonthermal atmospheric pressure plasma: A case series", JOURNAL OF THE AMERICAN ACADEMY OF DERMATOLOGY, vol. 76, no. 2, 13 January 2017 (2017-01-13), pages 349-350, and M WIRTZ ET AL: "Actinic keratoses treated with cold atmospheric plasma", J Eur Acad Dermatol Venereol . 2018 Jan;32(1):e37-e39.

In the presently described invention, it has been surprisingly found that CAP can be used in treating therapy-refractory actinic keratosis in a patient in need thereof, wherein the cold atmospheric plasma is an argon-containing plasma, with a high rate of success and no or essentially no adverse side effects.

### SUMMARY OF THE INVENTION

Actinic keratosis (AK) is the most frequent epidermal neoplasia that occurs in fair-skinned people with increased cumulative exposure to UV-radiation. In 2005, AK affected more than 58 million people in the USA with a reported prevalence of 11-26% according to recent reports. AK clinically appears as erythematous, hyperkeratotic, scaly or crusty macule, papule and plaque or, in its disseminated form, as field cancerization in chronically sun-exposed skin areas. Histologically, AK is characterized by intraepidermal keratinocytic dysplasia, which can transform into invasive squamous cell carcinoma (SCC) in up to 20% of the AK lesions within 10-25 years. SCC is a common form of Non-Melanoma Skin Cancer (NMSC). NMSC is the most common cancer occurring in people with fair skin and is a rising problem for health care services because it is more common than all other forms of cancer combined - with increasing incidence rates.

AK comprises pre-cancerous skin lesions, therefore selective and efficient therapy is essential to prevent neoplastic transformation. Depending on the clinical manifestation of AK and individual patient's characteristics, various therapy options are available and recommended in dermatological guidelines. Known therapies of AK used to prevent progress to NMSC include: topical treatments, such as self-application by the patient of cytostatic, immune-modifying or antineoplastic substances (e.g. diclofenac (sodium), ingenol mebutate, 5-fluorouracil, imiquimod (e.g. 3.75 or 5%) over a long specified period; photodynamic therapy, defined as selective destruction of tumor cells through application of photosensitization substance (e.g. aminolevulinic acid or methyl aminolevulinate (as hydrochloride)) followed by radiation with a special device (or daylight); and invasive treatments, such as cryotherapy, curettage, ablative laser or removal by surgical operation. All these known treatments are subject to side effects.

Topical treatments may produce serious side effects, including paresthesia, pain, toxic agranulocytosis, edema, bleeding, renal insufficiency, rash, local reactions/infections, chills, fever, arthralgia, nausea, headache, diarrhea, depression, alopecia, allergic reactions up to Stevens-Johnson-Syndrome.

Photodynamic therapy side effects include massive neuropathic pain (during therapy time), inflammatory pain, visual impairment up to blindness, edema, dermatitis solaris, hyperpigmentation, crust and scars. Side effects of invasive treatments include allergic reaction to anesthetics, pain, infections, bleeding, blistering, necrosis, wound healing disorder, hypo- or hyperpigmentation, scars and deforming cosmetic results.

There is a need for a non-invasive, simple and effective treatment for therapy-refractory AK that also has minimal side effects. It has now been surprisingly found that CAP can be used to treat therapy-refractory actinic keratosis in a patient in need thereof, wherein the cold atmospheric plasma is an argon-containing plasma, with a high rate of success and no or essentially no adverse side effects.

### BRIEF DESCRIPTION OF FIGURES

The patent or application file contains at least one drawing executed in color. Copies of this patent or patent application publication with color drawing(s) will be provided by the Office upon request and payment of the necessary fee. The Figure provides clinical images (patients 01, 02, 04, 06-2) showing actinic keratosis prior to (1^{st} column), during (after three CAP treatments, 2^{nd} column) and after seven CAP treatments (3^{rd} column).

### DETAILED DESCRIPTION

The scope of the present invention is defined by the appended claims.

The invention is directed to cold atmospheric plasma for use in treating therapy-refractory actinic keratosis in a patient in need thereof, wherein the cold atmospheric plasma is an argon-containing plasma.

Treating AK includes applying CAP to the affected area by using any CE-certified CAP device (e.g. Adtec SteriPlas by Adtec Plasma technology). The cold atmospheric plasma is an argon containing plasma, more particularly an argon containing plasma generated by microwaves. The plasma may be generated by an ionization chamber as described in WO 2007/031250. In the ionization chamber, argon gas is bombarded with electrons emitted from multiple hot electric filaments. The resulting plasma ions mix with air creating reactive agents to generate a wide uniform treatment field that is capable of treating larger tissue areas of about 4-900 cm². Unlike air-based plasmas that vary with temperature, pressure, and location, neutral argon plasma is a consistent and controllable energized gas with predictable active agents and constituents that include reactive oxygen and nitrogen species, OH radicals, ions, electrons, photons, UV light and activated argon species. Particularly, the cold atmospheric plasma has a comparatively low temperature, which is preferably below 100 °C, 75 °C or even 50 °C, more particularly 20 °C. The cold atmospheric plasma particularly has a temperature of 20-100 °C, more preferably 20-80 °C, particularly when measured at a distance of 20-100 mm from the ionization electrode.

Particularly, the device is configured such that the distance between the patients' affected areas to be treated with CAP and the ionization electrodes is controlled to be 2.5-30 cm, preferably 3-20 cm, most preferably 3.5-15 cm.

No tools, wound dressings, ointments or other procedures are necessary. Preferably treating AK includes regular CAP treatment of the AK affected area, similar to the treatment of chronic wounds. According to the invention, a patient affected with AK may be treated by exposing the affected area to cold atmospheric plasma for a time sufficient to induce an antitumoral response sufficient to destroy cancerous cells. The treatment may be repeated until the AK is sufficiently eliminated and clearance is achieved. Clearance means reduction of target lesions (there are less lesions after the treatment than at the beginning of the treatment (t=0) and/or improvement of the Olson grade (that is, the number of lesions are the same after the treatment, however the severity of the lesions is improved). Ideally clinically satisfactory clearance is reached, including complete clearance, that is no lesions after treatment. In an embodiment, for example, the duration of the application is preferably at least about two minutes, and is preferably repeated once or twice a week for several applications until the desired clearance is achieved.

In a preferred embodiment, the patient's affected area is exposed to the cold argon plasma according to the invention for 1-6 sessions a week, preferably two sessions a week. It is preferred that there is not more than one session a day. Thus, in another aspect, the patient's affected area is exposed to cold atmospheric plasma for one session a day, one session every two days, one session every four days, one session every five days or one session every six days.

In one aspect, the affected area is exposed to cold atmospheric plasma for 10-300 seconds per session, preferably 20-150 seconds per session, and most preferably 70-140 seconds.

In most cases, clearance is achieved after 5-30, preferably 5-20 sessions in total.

It was shown that the treatment according to the invention is particularly effective in patients who are fair-skinned (Fitzpatrick skin types I, II or III). The use according to the invention is effective in patients suffering from therapy-refractory actinic keratosis. Particularly patients suffering from actinic keratosis at an Olsen grade I, II or III, more particularly II or III, even more particularly II can effectively be treated by the use according to the invention. The use according to the invention may also be effective in patients suffering from field cancerization or untreated actinic keratosis.

In another aspect, the use according to the invention was shown to be effective in patients that were previously treated with diclofenac, particularly diclofenac in hyaluronic acid, ingenol mebutate, 5-fluorouracil and/or with imiquimod or photodynamic therapy, preferably using aminolevulinic acid, which treatment, however, did not result in a satisfactory clearance.

The use according to the invention may comprise a simultaneous or a consecutive treatment with an active agent against actinic keratosis, particularly with at least one of diclofenac (sodium), ingenol mebutate, 5-fluorouracil optionally together with salicylic acid, (methyl)aminolevulinic acid or salts thereof or imiquimod or photodynamic therapy, preferably using aminolevulinic acid or (methyl)aminolevulinate (e.g. as hydrochloride).

In another aspect, the use according to the invention is particularly effective in patients who are not simultaneously treated with an additional active agent against actinic keratosis during the treatment according to the invention.

Because AKs generally do not disappear immediately after CAP treatment, but the treatment induces an antitumoral response, a follow-up assessment should be conducted to ensure that the treatment has achieved clinically satisfactory clearance. Depending on the patient and condition, the application time, and repetition interval and number of applications needed to achieve the desired clearance may vary.

The use according to the invention has negligible side effects. While all established therapies cause local painful inflammatory or immune-modulating reactions - some even systemic side effects - CAP is completely painless and leaves healthy tissue unaffected since it is specific for tumor cells.

It was also shown that the use according to the present invention results in a continuous decline of erythema, scaling, crusts and thickness thereof, lesions and in a reduction of the total lesion number. Another interesting effect of CAP that was found is a potentially rejuvenating effect on the skin tone, lentigines, roughness and texture similar to that reported for other dermal remodeling physical procedures such as photodynamic therapy (PDT). A refined complexion, a reduction of hyper- or mottled pigmentation, and a reduction of hyperkeratosis and a palpatory firmer skin was observed in a subgroup of patients.

### EXAMPLES

### Example 1. Clinical Trial in Patients with Actinic Keratosis:

Seven patients (2 females, 5 males) with clinically diagnosed AK in their 50s to 80s were treated. All of them were fair-skinned with Fitzpatrick skin types II or III. All suffered from multiple, therapy-refractory AK or field-cancerization, mostly due to a history of extensive UV exposure (Table 1). One patient was under immune suppressive treatment with MTX 10 mg weekly because of psoriasis vulgaris et arthropathica.

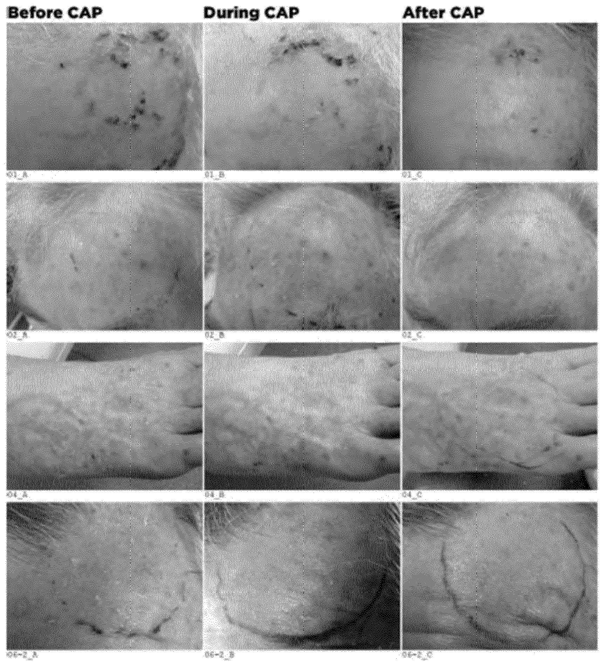

In each patient, a circular area of 19.64 cm², containing multiple therapy-refractory AK was defined, photo-documented (Fig 1, 1^{st} column), and the lesions were clinically classified according to Olsen grading: I slightly palpable, better felt than seen; II moderately thick, easily felt and seen; III very thick and hyperkeratotic. Seven CAP treatments were performed with a microwave-driven argon plasma jet (Adtec SteriPlas™ argon plasma (power consumption 1.5 kVA)) in each patient (twice a week, 120 seconds per treatment) after detailed explanation and obtaining informed consent.

During the CAP treatment period, no other concomitant therapy for AK was applied except standard physical UV protection procedures. Photo-documentation of the defined areas was done during and after seven CAP treatments (Fig 1, 2^{nd} and 3^{rd} columns).

A continuous decline of clinical AK characteristics such as erythema, scaling, crusts and thickness could be found in all eight treated areas (Table 2). In six areas, this resulted in clinical downgrading of the lesions according to Olsen grading and to a reduction of the total lesion number (Table 2). The other two treated areas showed limited total change from baseline, but a clinical Olsen downgrading of the existing lesions. A refined complexion with a discrete reduction of hyper- or mottled pigmentation seemed to occur in patients 3 and 7, a slightly firmer skin in patient 6 (site no. 1, tibia) and a reduction of hyperkeratosis in patients 3 and 4. No adverse events were reported and the treatment was well tolerated by all patients.

### Discussion of Clinical Results:

All patients treated in this case series showed promising responses after seven applications of CAP. Even though there are a variety of therapies for AK, CAP could become an important alternative for treatment because of its lack of side effects. In the study, no patient reported side effects, particularly no local pain or inflammation. Current physical (e.g. photodynamic therapy (PDT) or cryotherapy) and topical (e.g. imiquimod or ingenol mebutate) treatments for AK usually provoke local skin reactions such as erythema, flaking, scaling and crusting. Even for rather well tolerated topical agents, such as diclofenac 3% in 2.5% hyaluronic acid, dermatitis is a commonly reported side effect. Moreover, many local AK therapies such as fluorouracil or diclofenac cause photosensitivity, which limits their use in the summer.

The absence of side effects after CAP was, however, repeatedly documented also from numerous applications in chronic wound management where CAP is already frequently applied. Most likely, this allows CAP application also for larger body areas without a limit in the maximum treatment area, making it an attractive option especially for patients with extensively chronically sun-damaged skin or even immune-compromised patients. Other common topical therapies, such as 5% imiquimod, are limited to 25 cm².

Another interesting effect of CAP observed in a subgroup of patients is a potentially rejuvenating effect on the skin tone, lentigines, roughness and texture similar to that reported for other dermal remodeling physical procedures such as photodynamic therapy (PDT). A refined complexion, a reduction of mottled pigmentation and a firmer skin were observed in patients 3, 4, 6, and 7. The biological mode of action regarding the anti-tumorigenic potential of CAP remains unclear. Effects on the cell cycle and induction of senescence, apoptosis, and necrosis by reactive nitrogen and oxygen species have been discussed. Interestingly, the tumor-selectivity and apoptotic effect of CAP are especially high in p53-mutated cancer cells. Dysregulation of the p53 pathway plays an important role in inducing clonal keratinocytic expansion in AK and SCC. Since human papilloma viruses are considered co-carcinogenic in AK and SCC development and infected keratinocytes frequently express HPV oncoproteins, virocide properties of CAP might work synergistically.

In conclusion, even though there exists a variety of AK therapies, CAP might represent a novel and safe treatment because of its lack of side effects.

**Table 1: Patient overview (n=7)**

| **Patient** | **Sex** | **Age** | **Fitzpatrick skin type** | **UV-Exposure and special features** | **Prior history of skin cancer** | **Prior actinic keratosis treatments** | **Cold atmospheric plasma treatment area** |
|---|---|---|---|---|---|---|---|
| **01** | m | 82 | II | • increased exposure childhood | • squamous cell carcinoma (right shoulder, temporal right, left cheek lateral, left cheek medial, pectoral left, scapula left) | • Diclofenac 3% in 2.5% hyaluronic acid | • left forehead *(cf.* *fig. 1*/*01)* |
| | | | | | • actinic keratosis (flank left) | | |
| **02** | f | 82 | II | • increased exposure in holidays | • squamous cell carcinoma (pectoral left) | • Diclofenac 3% in 2.5% hyaluronic acid | • left forehead *(cf.* *fig. 1*/*02)* |
| | | | | | • M. Bowen (right abdomen, left forehead, nose) | • Photodynamic therapy | |
| **03** | m | 77 | III | • psoriasis vulgaris et arthropadiica (prior UV-therapy, MTX 10mg p.o.) | • melanoma (left upper arm) | • Diclofenac 3% in 2.5% hyaluronic acid | • dorsum left hand |
| | | | | | • lentigo maligna (right jaw angle) | • 5% Imiquimod | |
| | | | | | • basal cell carcinoma (nose) | | |
| | | | | | • M. Bowen (dorsum right hand) | | |
| | | | | | • actinic keratosis (2 x dorsum right hand) | | |
| **04** | f | 53 | II | • extensive use of tanning beds | • actinic keratosis (>12, various locations) | • Photodynamic therapy | • dorsum right foot *(cf.* *fig. 1*/*04)* |
| | | | | • father melanoma | | • 5% Imiquimod | |
| | | | | • mother actinic keratosis (capillitium, right malleolus medialis, Decolleté, dorsum of both hands, both lower legs) | • squamous cell carcinoma (>12, various locations) | | |
| | | | | | • superficial spreading melanoma (gluteal right) | | |
| | | | | • daughter melanoma | | | |
| **05** | m | 75 | II | • increased exposure in childhood and holidays | **-** | • Diclofenac 3% in 2.5% hyaluronic acid | • forehead |
| **06-1** | m | 77 | III | • increased exposure in childhood and holidays | • basal cell carcinoma (>25, various locations) | • Diclofenac 3% in 2.5% hyaluronic acid | • left tibia |
| | | | | | • melanoma (upper back) | • | |
| | | | | | | Cryotherapy • 5% Imiquimod | |
| **06-2** | " | " | " | " | " | " | • left forehead *(cf.* *fig. 1*/*06-2)* |
| **07** | m | 88 | II | - | • basal cell carcinoma (paravertebral left) | • Diclofenac 3% in 2.5% hyaluronic acid | • right forehead |

**Table 2: Lesion count and clinical observations**

| **Patient** | **Olsen Grade** | **No. at baseline** | **No. after treatment** | **Total change from baseline No. (%)** | **Clinical observations** |
|---|---|---|---|---|---|
| **01** | I | 4 | 14 | +10 (+250,00) | • massive haemorrhagic crusts at baseline, decrease during treatments *(cf.* *fig. 1*/*01)* |
| | II | 5 | 5 | 0 (0,00) | |
| | III | 19 | 1 | -18 (-94,74) | |
| | total | 28 | 20 | -8 (-28,57) | |
| **02** | I | 1 | 4 | +3 (+300,00) | • decrease of erythematous maculae and plaques *(cf.* *fig. 1*/*02)* |
| | II | 5 | 2 | -3 (-60,00) | |
| | III | 0 | 0 | 0 (0,00) | |
| | total | 6 | 6 | 0 (0,00) | |
| **03** | I | 3 | 6 | +3 (+100,00) | • refined complexion • reduction of hyperpigmentation • reduction of hyperkeratosis |
| | II | 8 | 7 | -1 (-12,50) | |
| | III | 2 | 0 | -2 (-100,00) | |
| | total | 13 | 13 | 0 (0,00) | |
| **04** | I | 8 | 22 | +14 (+175,00) | • reduction of hyperkeratosis |
| | II | 21 | 6 | -15 (-71,43) | • firmer complexion *(cf* *fig. 1*/*04)* |
| | III | 3 | 0 | -3 (-100,00) | |
| | total | 32 | 28 | -4 (-12,5) | |
| **05** | I | 2 | 7 | +5 (+250,00) | • reduction of hyperpigmentation |
| | II | 7 | 1 | -6 (-85,71) | |
| | III | 0 | 0 | 0 (0,00) | |
| | total | 9 | 8 | -1 (-11,11) | |
| **06-1** | I | 2 | 2 | 0 (0,00) | • firmer skin |
| | II | 4 | 2 | -2 (-50,00) | • reduction of scaling |
| | III | 1 | 0 | -1 (-100,00) | |
| | total | 7 | 4 | -3 (-42,86) | |
| **06-2** | I | 2 | 4 | +2 (+100,00) | • reduction of scaling *(cf.* *fig. 1*/*06-2)* |
| | II | 13 | 4 | -9 (-69,23) | |
| | III | 0 | 0 | 0 (0,00) | |
| | total | 15 | 8 | -7 (-46,67) | |
| **07** | I | 2 | 1 | -1 (-50,00) | • refined complexion |
| | II | 3 | 0 | -3 (-100,00) | • reduction of hyperpigmentation |
| | III | 0 | 0 | 0 (0,00) | • reduction of scaling |
| | total | 5 | 1 | -4 (-80,00) | |

## Claims

1. Cold atmospheric plasma for use in treating therapy-refractory actinic keratosis in a patient in need thereof, wherein the cold atmospheric plasma is an argon-containing plasma.

2. Cold atmospheric plasma according to claim 1 for use according to claim 1 by exposing the affected area to the cold atmospheric plasma and/or repeating said application until clearance is achieved.

3. Cold atmospheric plasma according to claim 1 for use according to claim 1 or 2, wherein the cold atmospheric plasma is an argon-containing plasma generated by microwaves.

4. Cold atmospheric plasma according to claim 1-2 for use according to any of claims 1-3, wherein the cold atmospheric plasma has a temperature of 20-100°C, preferably 20-40°C.

5. Cold atmospheric plasma according to claim 1-3 for use according to any of claims 1-4, wherein the affected area is exposed to cold atmospheric plasma for one to six sessions a week, preferably two sessions a week and/or for one session a day, one session every two days, one session every three days, one session every four days, one session every five days or one session every six days and/or for 5-30 sessions in total.

6. Cold atmospheric plasma according to any of the previous claims for use according to any of claims 1-5, wherein the affected area is exposed to the cold atmospheric plasma for 10-300 seconds per session.

7. Cold atmospheric plasma according to any of the previous claims for use according to any of claims 1-6, wherein the patient is fair-skinned and/or suffers from actinic keratosis Olsen grade I, II or III and/or suffers from field-cancerization.

8. Cold atmospheric plasma according to any of the previous claims for use according to any of claims 1-7, wherein the patient was previously treated with diclofenac or imiquimod or ingenol mebutate or 5-fluorouracil or 5-fluorouracil with salicylic acid.

9. Cold atmospheric plasma according to any of the previous claims for use according to any of claims 1-8, wherein the patient was previously treated with photodynamic therapy, preferably using (methyl)aminolevulinic acid or a salt thereof.

10. Cold atmospheric plasma according to any of the previous claims for use according to any of claims 1-9, wherein the treatment comprises a simultaneous treatment with at least one active agent selected from diclofenac, ingenol mebutate, 5-fluorouracil or imiquimod or a simultaneous photodynamic therapy, preferably using (methyl)aminolevulinic acid or a salt thereof.

11. Cold atmospheric plasma according to any of the previous claims for use according to any of claims 1-10, wherein the patient is not simultaneously treated with an additional active agent against actinic keratosis.

12. Cold atmospheric plasma according to any of the previous claims for use according to any of claims 1-11, wherein the cold atmospheric plasma application is applied for at least two minutes and is repeated once or twice a week until clearance is achieved.

## Patentansprüche

1. Kaltes atmosphärisches Plasma zur Verwendung bei der Behandlung von therapierefraktärer Aktinischer Keratose bei einem Patienten, der dies benötigt, wobei das kalte atmosphärische Plasma ein Argon-enthaltendes Plasma ist.

2. Kaltes atmosphärisches Plasma nach Anspruch 1 zur Verwendung nach Anspruch 1, indem der betroffene Bereich dem kalten atmosphärischen Plasma ausgesetzt und/oder die Anwendung wiederholt wird, bis eine Linderung erreicht ist.

3. Kaltes atmosphärisches Plasma nach Anspruch 1 zur Verwendung nach Anspruch 1 oder 2, wobei das kalte atmosphärische Plasma ein Argon-enthaltendes Plasma ist, welches mittels Mikrowellen generiert ist.

4. Kaltes atmosphärisches Plasma nach einem der Ansprüche 1-2 zur Verwendung nach einem der Ansprüche 1-3, wobei das kalte atmosphärische Plasma eine Temperatur von 20-100 °C, bevorzugt 20-40 °C aufweist.

5. Kaltes atmosphärisches Plasma nach einem der Ansprüche 1-3 zur Verwendung nach einer dem Ansprüche 1-4, wobei der betroffene Bereich dem kalten atmosphärischen Plasma für eine bis sechs Sitzungen pro Woche, bevorzugt zwei Sitzungen pro Woche und/oder für eine Sitzung pro Tag, eine Sitzung alle zwei Tage, eine Sitzung alle drei Tage, eine Sitzung alle vier Tage, eine Sitzung alle fünf Tage oder eine Sitzung alle sechs Tage und/oder für insgesamt 5-30 Sitzungen ausgesetzt wird.

6. Kaltes atmosphärische Plasma nach einem der vorhergehenden Ansprüche zur Verwendung nach einem der Ansprüche 1-5, wobei der betroffene Bereich dem kalten atmosphärischen Plasma für 10-300 Sekunden pro Sitzung ausgesetzt wird.

7. Kaltes atmosphärisches Plasma nach einem der vorhergehenden Ansprüche zur Verwendung nach einem der Ansprüche 1-6, wobei der Patient hellhäutig ist und/oder an Aktinischer Keratose mit Olsen-Grad I, II oder III leidet und/oder an Feldkanzerisierung leidet.

8. Kaltes atmosphärische Plasma nach einem der vorhergehenden Ansprüche zur Verwendung nach einem der Ansprüche 1-7, wobei der Patient zuvor mit Diclofenac oder Imiquimod oder Ingenol-Mebutat oder 5-Fluorouracil oder 5-Fluorouracil mit Salicylsäure behandelt worden ist.

9. Kaltes atmosphärische Plasma nach einem der vorhergehenden Ansprüche zur Verwendung nach einem der Ansprüche 1-8, wobei der Patient zuvor mit photodynamischer Therapie, bevorzugt unter Verwendung von (Methyl)aminolävulin-Säure oder einem Salz davon, behandelt worden ist.

10. Kaltes atmosphärisches Plasma nach einem der vorhergehenden Ansprüche zur Verwendung nach einem der Ansprüche 1-9, wobei die Behandlung eine gleichzeitige Behandlung mit wenigstens einem Wirkstoff, ausgewählt aus Diclofenac, Ingenol-Mebutat, 5-Fluorouracil oder Imiquimod oder eine gleichzeitige photodynamische Therapie, bevorzugt unter Verwendung von (Methyl)aminolävulin-Säure oder einem Salz davon, umfasst.

11. Kaltes atmosphärisches Plasma nach einem der vorhergehenden Ansprüche zur Verwendung nach einem der Ansprüche 1-10, wobei der Patient nicht gleichzeitig mit einem zusätzlichen Wirkstoff gegen Aktinische Keratose behandelt wird.

12. Kaltes atmosphärisches Plasma nach einem der vorhergehenden Ansprüche zur Verwendung nach einem der Ansprüche 1-11, wobei die Anwendung mit kaltem atmosphärischen Plasma für wenigstens zwei Minuten angewendet wird und ein Mal oder zwei Mal pro Woche wiederholt wird, bis eine Linderung erreicht wird.

## Revendications

1. Plasma froid atmosphérique destiné à être utilisé dans le traitement d'une kératose actinique réfractaire aux autres thérapies chez un patient ayant besoin d'un tel traitement, dans lequel le plasma froid atmosphérique est un plasma contenant de l'argon.

2. Plasma froid atmosphérique selon la revendication 1 destiné à être utilisé selon la revendication 1 en exposant la zone touchée au plasma froid atmosphérique et/ou en répétant ladite application jusqu'à la disparition des lésions.

3. Plasma froid atmosphérique selon la revendication 1 destiné à être utilisé selon la revendication 1 ou 2, dans lequel le plasma froid atmosphérique est un plasma contenant de l'argon généré par des micro-ondes.

4. Plasma froid atmosphérique selon la revendication 1 à 2 destiné à être utilisé selon l'une quelconque des revendications 1 à 3, dans lequel le plasma froid atmosphérique a une température allant de 20 à 100 °C, de préférence de 20 à 40 °C.

5. Plasma froid atmosphérique selon la revendication 1 à 3 destiné à être utilisé selon l'une quelconque des revendications 1 à 4, dans lequel la zone touchée est exposée au plasma froid atmosphérique pendant une à six séances par semaine, de préférence deux séances par semaine et/ou pendant une séance par jour, une séance tous les deux jours, une séance tous les trois jours, une séance tous les quatre jours, une séance tous les cinq jours ou une séance tous les six jours et/ou pendant 5 à 30 séances au total.

6. Plasma froid atmosphérique selon l'une quelconque des revendications précédentes destiné à être utilisé selon l'une quelconque des revendications 1 à 5, dans lequel la zone touchée est exposée au plasma froid atmosphérique pendant 10 à 300 secondes par séance.

7. Plasma froid atmosphérique selon l'une quelconque des revendications précédentes destiné à être utilisé selon l'une quelconque des revendications 1 à 6, dans lequel le patient a la peau claire et/ou souffre d'une kératose actinique de grade Olsen I, II ou II et/ou souffre d'une cancérisation en champ.

8. Plasma froid atmosphérique selon l'une quelconque des revendications précédentes destiné à être utilisé selon l'une quelconque des revendications 1 à 7, dans lequel le patient a précédemment été traité avec du diclofénac ou de l'imiquimod ou du mébutate d'ingénol ou du 5-fluorouracile ou du 5-fluorouracile avec de l'acide salicylique.

9. Plasma froid atmosphérique selon l'une quelconque des revendications précédentes destiné à être utilisé selon l'une quelconque des revendications 1 à 8, dans lequel le patient était précédemment traité par thérapie photodynamique, de préférence en utilisant de l'acide méthyl-aminolévulinique ou un sel de celui-ci.

10. Plasma froid atmosphérique selon l'une quelconque des revendications précédentes destiné à être utilisé selon l'une quelconque des revendications 1 à 9, dans lequel le traitement comprend un traitement simultané avec au moins un agent actif sélectionné parmi le diclofénac, le mébutate d'ingénol, le 5-fluorouracile ou l'imiquimod ou une thérapie photodynamique simultanée, de préférence en utilisant de l'acide méthyl-aminolévulinique ou un sel de celui-ci.

11. Plasma froid atmosphérique selon l'une quelconque des revendications précédentes destiné à être utilisé selon l'une quelconque des revendications 1 à 10, dans lequel le patient n'est pas simultanément traité avec un autre agent actif contre la kératose actinique.

12. Plasma froid atmosphérique selon l'une quelconque des revendications précédentes destiné à être utilisé selon l'une quelconque des revendications 1 à 11, dans lequel l'application de plasma froid atmosphérique est appliquée pendant au moins deux minutes et est répétée une ou deux fois par semaine jusqu'à la disparition des lésions.
